# EUROPEAN PATENT APPLICATION

(11) **EP 4 656 713 A1**
(43) Date of publication of application: **03.12.2025**
(21) Application number: 24747312.7
(22) Date of filing: 23.01.2024
(51) Int. Cl.: C12N 5/00, C12N 1/00, C12N 5/10, C12N 5/074, C12N 5/0735

(54) **CELL AGGREGATE DISSOCIATING AGENT**

(30) Priority: 24.01.2023 JP 2023008929
(71) Applicant: AJINOMOTO CO., INC., Chuo-ku Tokyo 104-8315 (JP)
(72) Inventor: SUDO SUZUKI, Yu, Kawasaki-shi, Kanagawa 210-8681 (JP); HIGUCHI, Takuya, Kawasaki-shi, Kanagawa 210-8681 (JP); MIYAIRI, Kyohei, Kawasaki-shi, Kanagawa 210-8681 (JP); ITO, Kenichiro, Kawasaki-shi, Kanagawa 210-8681 (JP)
(74) Representative: Hoffmann Eitle
(86) International application number: PCT/JP2024/001907
(87) International publication number: WO 2024/157985

(57) **Abstract**

The present invention aims to provide a method for dissociating cell aggregates, which are formed in cell culture, particularly in the cell passaging step, more easily and with less damage to cells, a method for culturing cells using said method, and a cell aggregate dissociation agent therefor. The present invention relates to a method for dissociating cell aggregates, including subjecting cell aggregates to a treatment with a water-insoluble metal ion adsorbent.

## Description

### [Technical Field]

The present invention relates to a cell culture technique, particularly, a technique for dissociating cell aggregates formed during cell culture.

### [Background Art]

In order to efficiently culture large quantities of cells in cell culture, particularly in animal cell culture, the technical development of suspension culture (also called 3D culture) is underway. Since suspension culture does not require a culture surface, such as the one in adherent culture using a petri dish and the like, it shows high productivity per installation area, is also superior in scalability, and thus more suitable for mass production. Particularly in regenerative medicine, the technical development for suspension culture of pluripotent stem cells such as iPS cells is indispensable for industrialization thereof.

Various studies have been conducted as to culture steps toward large-scale cell production and they are maturing, but the passage step is under development. In suspension culture, cells are generally cultured in a bioreactor, and a step is performed to form aggregates called spheroids consisting of several hundred cells. During the culture process, aggregates of cells are once formed, but passaging requires multiple steps of separating the cell aggregates from the medium, washing, adding a dissociation agent for the purpose of forming single cells or clumping and reaction, separating the dissociation agent from the formed single cells or clumped cells, and resuspending the cells in a culture medium, and they are extremely complicated.

For example, iPS cells form cell aggregates when cultured in suspension, but the cell aggregates need to be dissociated into single cells or clumped to perform passage. A method of adding a solution of a low molecular weight chelating agent (EDTA, etc.) or an enzyme, as a dissociation agent for forming single cells, to the aggregates is generally known, in which the dissociation agent needs to be removed by centrifugation after the reaction (Non Patent Literature 1). In addition, a method of physically dissociating cell aggregates by a dissociation force generated in an agitation reactor has also been reported (Patent Literature 1).

On the other hand, a method of adding an ion exchange resin in culturing adherent cells has been reported (Non Patent Literature 1), but this is use as a pH buffering agent during culture and not for the purpose of dissociating the cells.

### [Citation List]

### [Patent Literature]

[Patent Literature 1]
WO2017127921A1

### [Non Patent Literature]

[Non Patent Literature 1]
   J. Beers et al., Nat Protoc. 2012; 7(11): 2029-2040.
[Non Patent Literature 2]
   T. Matsumura et al., Experimental Cell Research, Vol 53, Issues 2-3, 1968, pp. 337-347

### [Summary of Invention]

### [Technical Problem]

The present invention aims to provide a method for dissociating cell aggregates more easily and with less damage to cells, in cell culture, particularly in the cell passaging step, a method for culturing cells using said method, and an agent therefor.

### [Solution to Problem]

In view of the above-mentioned problem, the present inventors have found that an ion exchange resin, used as a cell aggregate dissociation agent instead of a low-molecular-weight chelating agent, can be removed without centrifugation of the dissociation agent after dissociation, and confirmed that cells dissociated in this way are less susceptible to damage and can be passaged, which resulted in the completion of the present invention.

Accordingly, the present invention provides the following.

[1] A method for dissociating a cell aggregate, comprising subjecting the cell aggregate to a treatment with a water-insoluble metal ion adsorbent in a vehicle.
[2] The method of the above-mentioned [1], wherein the metal ion adsorbent has sedimentation property.
[3] The method of the above-mentioned [1] or [2], wherein the metal ion adsorbent is housed in a member impermeable to metal ion adsorbents.
[4] A method for culturing cells, comprising the following steps:
   a first step of preparing a cell aggregate;
   a second step of dissociating the cell aggregate obtained in the first step by a treatment with a water-insoluble metal ion adsorbent in a vehicle;
   and a third step of culturing the dissociated cells obtained in the second step.
[5] The method of the above-mentioned [4], wherein the metal ion adsorbent has sedimentation property.
[6] The method of the above-mentioned [4] or [5], wherein the metal ion adsorbent is housed in a member impermeable to metal ion adsorbents.
[7] The method of any of the above-mentioned [1] to [6], wherein the metal ion adsorbent has cation exchange property.
[8] The method of any of the above-mentioned [1] to [7], wherein the metal ion adsorbent is a weakly acidic cation exchange resin.
[9] The method of any of the above-mentioned [1] to [8], wherein the metal ion is Ca²⁺ and/or Mg²⁺.
[10] The method of any of the above-mentioned [1] to [9], wherein the cell is a stem cell or a progenitor cell.
[11] The method of the above-mentioned [10], wherein the stem cell is a pluripotent stem cell or an adult stem cell.
[12] The method of the above-mentioned [11], wherein the pluripotent stem cell is an induced pluripotent stem cell (iPS cell) or an embryonic stem cell (ES cell).
[13] The method of the above-mentioned [11], wherein the adult stem cell is a mesenchymal stem cell.
[14] The method of any of the above-mentioned [4] to [13], wherein the first step is performed under adherent culture conditions.
[15] The method of any of the above-mentioned [4] to [13], wherein the first step is performed under suspension culture conditions.
[16] The method of any of the above-mentioned [4] to [15], wherein the second step is performed under adherent culture conditions.
[17] The method of any of the above-mentioned [4] to [15], wherein the second step is performed under suspension culture conditions.
[18] The method of any of the above-mentioned [4] to [17], wherein the third step is performed under adherent culture conditions.
[19] The method of any of the above-mentioned [4] to [17], wherein the third step is performed under suspension culture conditions.
[20] The method of any of the above-mentioned [1] to [19], wherein cell aggregate dissociation is inhibition of cell-cell adhesion.
[21] The method of any of the above-mentioned [1] to [19], wherein cell aggregate dissociation is inhibition of cell-substrate adhesion.
[22] A cell aggregate dissociation agent comprising a water-insoluble metal ion adsorbent.
[23] The dissociation agent of the above-mentioned [22], wherein the metal ion adsorbent has sedimentation property.
[24] The dissociation agent of the above-mentioned [22] or [23], wherein the metal ion adsorbent is housed in a member impermeable to metal ion adsorbents.
[25] The dissociation agent of any of the above-mentioned [22] to [24], wherein the metal ion adsorbent has cation exchange property.
[26] The dissociation agent of any of the above-mentioned [22] to [25], wherein the metal ion adsorbent is a weakly acidic cation exchange resin.
[27] The dissociation agent of any of the above-mentioned [22] to [26], wherein the metal ion is Ca²⁺ and/or Mg²⁺.
[28] The dissociation agent of any of the above-mentioned [22] to [27], wherein the cell is a stem cell or a progenitor cell.
[29] The dissociation agent of the above-mentioned [28], wherein the stem cell is a pluripotent stem cell or an adult stem cell.
[30] The dissociation agent of the above-mentioned [29], wherein the pluripotent stem cell is an induced pluripotent stem cell (iPS cell) or an embryonic stem cell (ES cell).
[31] The dissociation agent of the above-mentioned [29], wherein the adult stem cell is a mesenchymal stem cell.

### [Advantageous Effects of Invention]

According to the present invention, cell aggregates, particularly cell aggregates suspended in culture medium, can be efficiently dissociated, and the dissociated cells can be collected without a centrifugation treatment and can be suitably used for passaging. As a result, more cells, particularly pluripotent stem cells such as iPS cells, can be obtained, and the cells can be supplied in large quantities for use in research, medicine, and the like.

### [Brief Description of Drawings]

[Fig. 1]

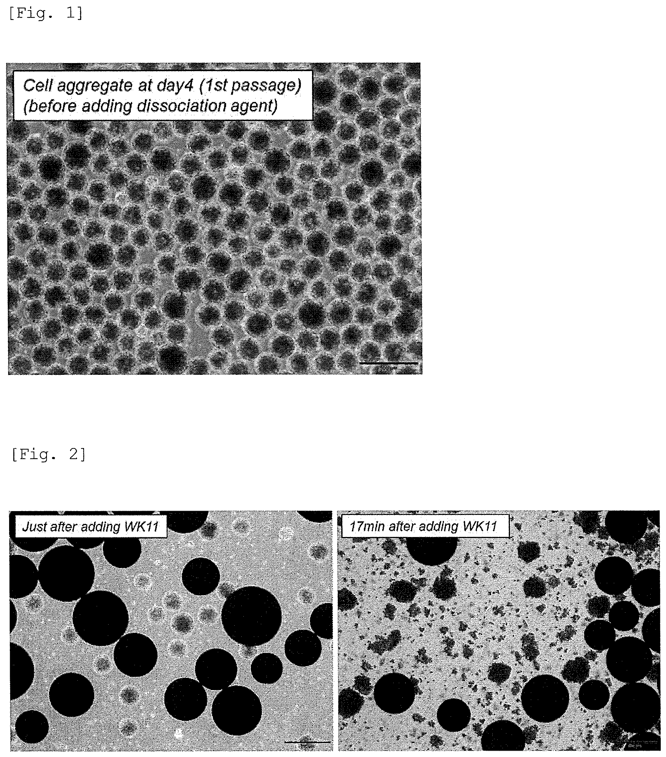

   Fig. 1 is a micrograph of the cell aggregates after 4 days of suspension culture in Example 1(3). Scale bar: 500 µm.
[Fig. 2]
   Fig. 2 is a micrograph of the state of dissociation of cell aggregates by WK11 in Example 1(4). The left is a micrograph taken just after adding WK11, and the right is a micrograph taken after adding WK11 and stirring for 17 min. Scale bar: 500 µm.
[Fig. 3]

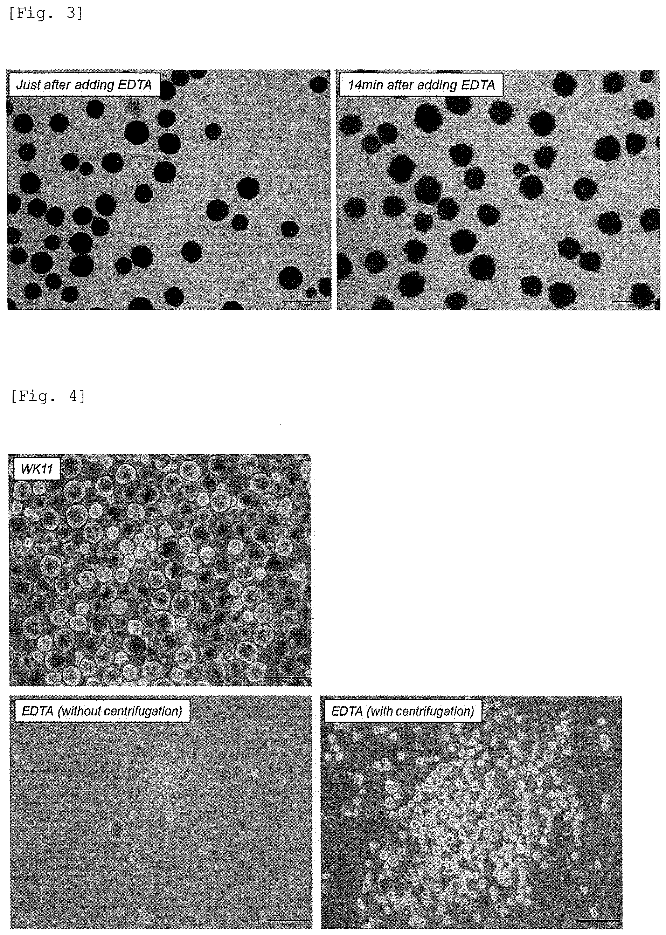

   Fig. 3 is a micrograph showing the state of dissociation of cell aggregates by EDTA in Example 1(5) and (6). The left is a micrograph taken just after adding EDTA, and the right is a micrograph taken after adding EDTA and stirring for 14 min. Scale bar: 500 µm.
[Fig. 4]
   Fig. 4 is a micrograph showing the state of cells dissociated under the following conditions (1st Passage) and suspension cultured for 4 days. Condition 1: Dissociation of cell aggregates by WK11 (top image), Condition 2: Dissociation of cell aggregates by EDTA (bottom left image), Condition 3: Dissociation of cell aggregates by EDTA → centrifugation (bottom right image).
[Fig. 5]

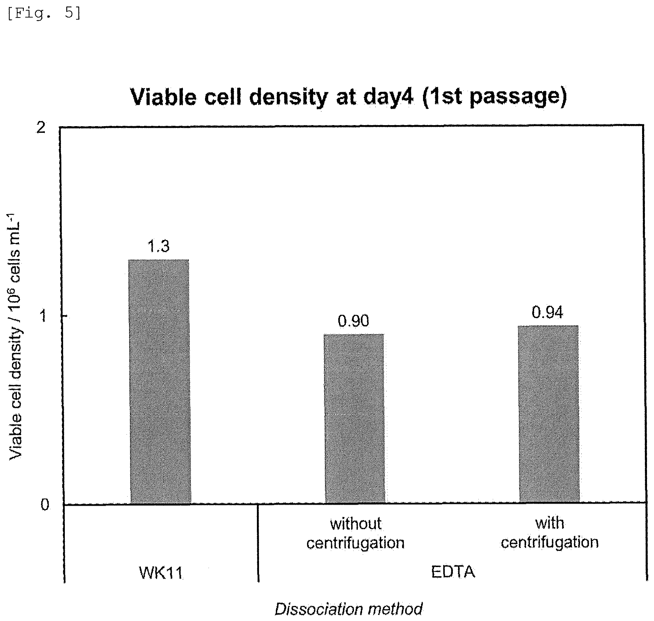

   Fig. 5 is a graph showing the measurement results of the viable cell density of cells dissociated under the following conditions (1st Passage) and suspension cultured for 4 days (Condition 1, Condition 2, Condition 3 from the left). Condition 1: Dissociation of cell aggregates by WK11, Condition 2: Dissociation of cell aggregates by EDTA, Condition 3: Dissociation of cell aggregates by EDTA → centrifugation.
[Fig. 6]

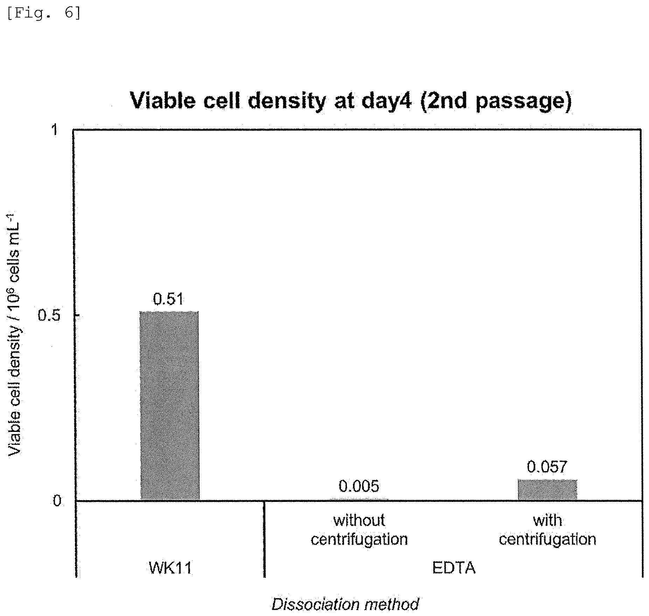

   Fig. 6 is a graph showing the measurement results of the viable cell density of cells dissociated under the following conditions, suspension cultured for 4 days, then reseeded (2nd Passage), and suspension cultured for additional 4 days (Condition 1, Condition 2, Condition 3 from the left). Condition 1: Dissociation of cell aggregates by WK11, Condition 2: Dissociation of cell aggregates by EDTA, Condition 3: Dissociation of cell aggregates by EDTA → centrifugation.
[Fig. 7]

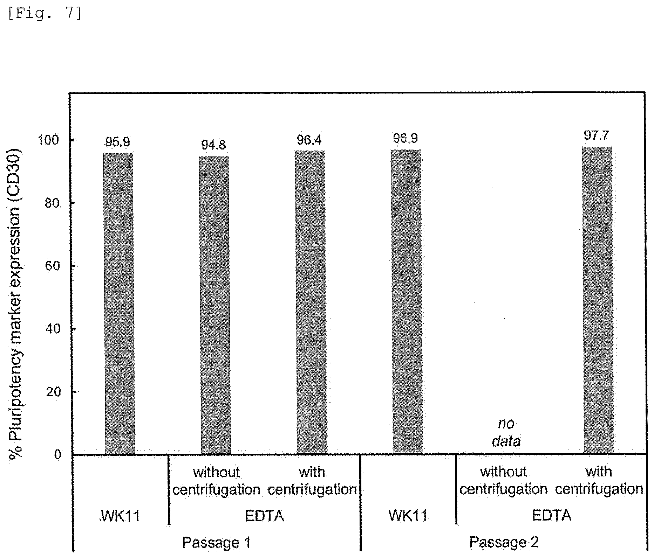

   Fig. 7 is a graph showing the evaluation results of the undifferentiated rate of cells at 4 days after suspension culture seeding in Passage 1 and Passage 2, using CD30 expression as an index (Condition 1, Condition 2, and Condition 3 from the left in both Passage 1 and Passage 2). Cell dissociation was performed under the following conditions. Condition 1: Dissociation of cell aggregates by WK11, Condition 2: Dissociation of cell aggregates by EDTA, Condition 3: Dissociation of cell aggregates by EDTA → centrifugation.

### [Description of Embodiments]

The present invention is explained below. The terms used in the present specification have the meanings generally used in the relevant field unless particularly specified.

### 1. Cell aggregate dissociation agent

The present invention provides an agent for dissociating cell aggregates (hereinafter also to be referred to as the "cell aggregate dissociation agent of the present invention" or the "dissociation agent of the present invention"), which is characterized by containing a water-insoluble metal ion adsorbent.

### (Cell aggregate)

In the present invention, a "cell aggregate" is also called a spheroid or sphere, and is a mass of cells in which several dozens to several hundreds of cells are aggregated, and generally has a spherical shape. Here, "spherical" includes a nearly spherical shape such as an egg shape or a rugby ball shape, in addition to a completely spherical shape. In addition to a population of cells adhering to each other in a nearly spherical shape referred to as spheroid in the present specification, the cell aggregate also includes an adherent cell population obtained by cells growing while adhering to the surface of the culture substrate, a colony formed by cells closely adhering to each other, and the like. Organoids consisting of multiple cells are also included. In suspension culture, when the cell aggregate exceeds a certain size due to cell aggregation, a passaging step is required because the growth rate decreases and the cell quality deteriorates.

### (Metal ion adsorbent)

In the present invention, the "metal ion adsorbent" refers to a material that can adsorb metal ions necessary for cell adhesion in a culture medium and remove them from the cell culture environment, thereby cutting off cell adhesion and dissociating cell aggregates into single cells. As used herein, "cell adhesion" refers to both "cell-cell adhesion" necessary for cell aggregation under suspension culture conditions, and "cell-substrate adhesion" necessary for cell aggregation under adherent culture conditions.

Cell adhesion requires the action of adhesive proteins such as cadherin (e.g., E-cadherin, N-cadherin), integrin and the like. For example, cadherin has an action to promote cell-cell adhesion in a calcium ion-dependent manner, and an action to promote cell-substrate adhesion in a magnesium ion-dependent manner. Therefore, in the present invention, the metal ion to be adsorbed is a metal ion required by adhesive proteins, and preferably includes calcium ion (Ca²⁺) and/or magnesium ion (Mg²⁺). The metal ion adsorbent of the present invention is preferably a cation-exchangeable substance having a negatively charged functional group (cation exchange group) since it can form a complex with these Ca²⁺ and Mg²⁺. As cation exchange group, sulfonic acid group, carboxylic acid group, phosphate group, phenolic hydroxyl group, sulfate ester group, phosphate ester group, and the like are known. Furthermore, the metal ion adsorbent of the present invention is preferably insoluble in water since recovery after treatment is easy and, for example, it is preferably in an embodiment in which the cation exchange group is bound to a water-insoluble substrate. When the metal ion adsorbent or the dissociation agent of the present invention containing the same is insoluble in water, the dissociated cells (single-celled cells) can be separated from the metal ion adsorbent or the dissociation agent of the present invention by simply allowing them to stand without any special treatment such as centrifugation.

The water-insoluble substrate is not particularly limited as long as it can be used in a cell culture environment, and any solid material can be used. Suitable substrates include, but are not limited to, synthetic resins, gold particles, magnetic particles, membranes, fibers, thin metal films, and the like. The shape is preferably bead-like (particulate).

A preferred metal ion adsorbent is a resin bonded to a cation exchange group, known as a cation exchange resin. Cation exchange resins are capable of exchanging cations in an aqueous solution for another type of cation, are used in water treatment for producing pure water and soft water, purification of pharmaceutical products and foods, and the like, and can also be used in the present invention.

Cation exchange resins are synthetic resins (base material) that have cation exchange groups, and are generally 20-50 mesh spherical or amorphous, with the backbone polymer being insolubilized by crosslinking. Depending on the acidity of the cation exchange group, they are divided into strongly acidic cation exchange resin and weakly acidic cation exchange resin.

Examples of the strongly acidic cation exchange resin include resins whose exchange group is a sulfone group. Specific examples thereof include Diaion SK1B, SK104, SK110, SK112, SK116, PK208, PK212, PK216, PK220, PK228, and HPK25 (all trade names) manufactured by Mitsubishi Chemical Corporation, and Amberlite IR-120B, IR-124, 200C, 201B, 252, and IR-118 (all trade names) manufactured by ORGANO CORPORATION. Examples of the weakly acidic cation exchange resin include methacrylic acid-based and acrylic acid-based ion exchange resins whose exchange group is a carboxylic acid group. Specific examples thereof include Diaion WK10, WK11, WK100, WT01S, and WK40 (all trade names) manufactured by Mitsubishi Chemical Corporation, and Amberlite IRC-50 and IRC-76 (all trade names) manufactured by ORGANO CORPORATION. In the present invention, the metal ion adsorbent is a water-insoluble, preferably sedimentary weakly acidic cation exchange resin.

### (Cells)

The type of cells targeted in the present invention is not particularly limited as long as the cells form a cell aggregate and are desired to be single-celled by dissociating the aggregate. Examples of such cell type include a somatic cell that forms a living organism, a stem cell, a progenitor cell, a cancer cell isolated from a living organism, a cell (cell line) that has been isolated from a living organism, has acquired immortality ability, and is stably maintained outside the body, a cell that has been isolated from a living organism and artificially modified genetically, a cell that has been isolated from a living organism and has artificially exchanged nuclei, and the like. Examples of somatic cells that form a living organism include, but are not limited to, fibroblast, bone marrow cell, B lymphocyte, T lymphocyte, neutrophil, erythrocyte, platelet, macrophage, monocyte, osteocyte, pericyte, dendritic cell, keratinocyte, adipocyte, mesenchymal cell, epithelial cell, epidermal cell, endothelial cell, vascular endothelial cell, hepatic parenchymal cell, chondrocyte, cumulus cell, neural cell, glial cell, neuron, oligodendrocyte, microglia, astrocyte, heart cell, esophageal cell, muscle cells (e.g., smooth muscle cell or skeletal muscle cell), pancreatic beta cell, melanocyte, hematopoietic progenitor cell (e.g., CD34 positive cell derived from umbilical cord blood), mononuclear cell, and the like. The somatic cell includes cells taken from any tissue, such as skin, kidney, spleen, adrenal gland, liver, lung, ovary, pancreas, uterus, stomach, colon, small intestine, large intestine, bladder, prostate, testis, thymus, muscle, connective tissue, bone, cartilage, vascular tissue, blood (including umbilical cord blood), bone marrow, heart, eye, brain, or nerve tissue.

Preferably, the cell is a stem cell or a progenitor cell.

Stem cells are cells that have the ability to replicate themselves and the ability to differentiate into cells of other multiple lineages. Stem cells include subpopulations such as pluripotent stem cells, multipotent stem cells, and unipotent stem cells, depending on the differentiation ability.

Multipotent stem cells refer to stem cells that have the ability to differentiate into multiple types of tissues or cells, though not all types, and unipotent stem cells refer to stem cells that have the ability to differentiate into specific tissues or cells. As multipotent or unipotent stem cells, adult stem cells (also called somatic stem cells or tissue stem cells) are known. Specifically, they include neural stem cells, hematopoietic stem cells, mesenchymal stem cells, hepatic stem cells, pancreatic stem cells, muscle stem cells, germline stem cells, intestinal stem cells, cancer stem cells, hair follicle stem cells, and the like.

Pluripotent stem cells are stem cells that can be cultured in vitro and have the ability to differentiate into all cell lineages belonging to the three germ layers (ectoderm, mesoderm, endoderm). As pluripotent stem cells, embryonic stem cells (ES cells), embryonic tumor cells (EC cells), embryonic germ stem cells (EG cells), nuclear transfer ES cells, somatic cell-derived ES cells (ntES cells), and induced pluripotent stem cells (iPS cells) are known. Pluripotent stem cells that are induced and selected by stress or cell stimulation, and the like can be mentioned. Stem cells established by culturing early embryos produced by nuclear transfer of somatic cell nuclei can also be used as the pluripotent stem cells (Nature, 385, 810 (1997); Science, 280, 1256 (1998); Nature Biotechnology, 17, 456 (1999); Nature, 394, 369 (1998); Nature Genetics, 22, 127 (1999); Proc. Natl. Acad. Sci. USA, 96, 14984 (1999); Nature Genetics, 24, 109 (2000)). In the present invention, iPS cells are preferred as pluripotent stem cells. Identification of iPS cells can be performed using undifferentiated markers due to the undifferentiated properties of iPS cells as indicators. Examples of the undifferentiation marker include alkaline phosphatase, Oct3/4, Sox2, Nanog, ERas, Esgl, and the like. In addition, CD30 can be used as an undifferentiation marker according to a previous report (M. A. Lagarkova et al., Cell Cycle, 7, 3610-3612 (2008)). Examples of the method for detecting these undifferentiation markers include methods for detecting mRNA (using primers and probes), immunological detection methods (using antibodies and labels), and the like.

"Progenitor cells" refer to cells that can develop from stem cells and differentiate into terminally differentiated cells that constitute the body. Stem cells differentiate into terminally differentiated cells via progenitor cells, and thus the progenitor cells can be considered as cells that are intermediate between stem cells and terminally differentiated cells.

"Cell line" refers to cells that have acquired infinite proliferation potential through artificial manipulation in vitro. A cell line is a cell that has acquired infinite proliferation potential through artificial manipulation in vitro, and examples thereof include, but are not limited to, CHO (Chinese hamster ovary cell line), HCT116, Huh7, HEK293 (human embryonic kidney cell), HeLa (human uterine cancer cell line), HepG2 (human liver cancer cell line), UT7/TPO (human leukemia cell line), MDCK, MDBK, BHK, C-33A, HT-29, AE-1, 3D9, Ns0/1, Jurkat, NIH3T3, PC12, S2, Sf9, Sf21, High Five (registered trademark), Vero, and the like.

In one preferred embodiment, the cell is a stem cell or progenitor cell, more preferably may be a pluripotent stem cell, particularly an iPS cell or ES cell, or may be an adult stem cell, particularly a mesenchymal stem cell.

### 2. Dissociation method for cell aggregates

The present invention provides a method for dissociating cell aggregates (hereinafter also to be referred to as the "dissociation method of the present invention"), and the method is characterized by subjecting cell aggregates to a treatment with a water-insoluble metal ion adsorbent.

The cells to be the target of the dissociation method of the present invention can be the same as those described in the above-mentioned "1. Cell aggregate dissociation agent", and preferred embodiments are also the same.

The "metal ion adsorbent" to be used in the dissociation method of the present invention can be the same as those described in the above-mentioned "1. Cell aggregate dissociation agent", and preferred embodiments are also the same.

In the present invention, the embodiment of the "treatment with a metal ion adsorbent" is not particularly limited as long as the cell aggregates and the metal ion adsorbent can exist in the same vehicle for a certain period of time. For example, when the treatment is performed under suspension culture conditions, a batch method, a column method, or a combination thereof can be used. When the treatment is performed under adherent culture conditions, addition of a metal ion adsorbent (or the cell aggregate dissociation agent of the present invention containing the same) to the cell aggregates on the substrate, and the like can be mentioned. The amount of the metal ion adsorbent to be used is not particularly limited as long as it can reduce the concentration of metal ion in the vehicle. The degree of reduction of the metal ion concentration should be sufficient to dissociate the cell aggregates, and is appropriately set according to the size of the cell aggregates to be dissociated, the type of cells constituting same, the treatment conditions, and the like. In one embodiment, the concentration of Ca²⁺ in the vehicle is 100 µM or less, 90 µM or less, 80 µM or less, 70 µM or less, 60 µM or less, 50 µM or less, 40 µM or less, 30 µM or less, or 20 µM or less.

For example, when a cation exchange resin is used as the metal ion adsorbent in the batch method, the cation exchange resin is added to the vehicle (e.g., culture medium) containing the cell aggregates, they are generally stirred for 5 min to 1 hr, preferably 10 to 20 min, then loosened by pipetting or the like as desired, after which the cation exchange resin is separated. The separation of the cation exchange resin can be performed by a conventional method such as filtration, centrifugation and the like. In the present invention, a water-insoluble sedimentary cation exchange resin (metal ion adsorbent) is used, and the cation exchange resin can be sedimented and easily separated from single-celled cells by simply allowing the resin to stand for several seconds to several tens of minutes, preferably within 30 min, more preferably within 10 min, without performing filtration or centrifugation.

Alternatively, by housing the metal ion adsorbent in a member impermeable to the metal ion adsorbent and immersing the member in a vehicle (e.g., culture medium) containing the cell aggregates, the cell aggregates and the metal ion adsorbent can exist in the same vehicle for a certain period of time in a state in which they are isolated from each other. The member that is impermeable to the metal ion adsorbent is not particularly limited. When the metal ion adsorbent is in the form of beads, a membrane or mesh made of a porous material with a pore size smaller than the diameter of the beads, a device equipped with such a membrane or mesh, and the like can be mentioned. The material of the porous material is not particularly limited as long as it does not adversely affect the cells, and it may be an organic compound, an inorganic compound, or a combination of these. Examples of the organic compound include polymer compounds such as polylactic acid (PLA), poly(vinyl alcohol) (PVA), gelatin, collagen, hyaluronic acid, polylactic acid-glycolic acid copolymer (PLGA), polycaprolactone (PCL), chondroitin sulfate, chitin, chitosan, cellulose, alginic acid, fibronectin, laminin, polyethylene and the like, and the like. Examples of the inorganic compound include metals such as titanium, gold, silver, nickel, nickel-chrome alloy, stainless steel and the like; oxides of these metals; hydroxyapatite, zirconia, alumina, calcium phosphate and the like. By housing the metal ion adsorbent in a member with a smaller pore size than the metal ion adsorbent, the metal ion adsorbent is prevented from diffusing into the vehicle, and the metal ion adsorbent after treatment is easily recovered. By using a membrane, mesh, and the like, the metal ion adsorbent such as a cation exchange resin can be separated from the cells, without being left to stand. In order to facilitate recovery of the metal ion adsorbent after treatment, a certain amount of the metal ion adsorbent may be solidified and molded into a bulk shape. The "bulk" refers to a three-dimensional shape having a certain thickness, such as a plate or a block. The bulk metal ion adsorbent can be used by immersing same in a vehicle containing cell aggregates. Even when the metal ion adsorbent itself does not have sedimentation property, it can be made to have sufficient sedimentation property by molding into a bulk shape.

The column type may be either a circulation method or a flow method. The circulation method is a method in which a vehicle containing cell aggregates (e.g., culture medium) is passed many times through a column filled with a cation exchange resin. The flow method is a method in which a vehicle (e.g., culture medium) containing cell aggregates is passed only once through a column. Since sufficient single cell dissociation is required when a column type is adopted, the circulation method is preferred.

In the present invention, the metal ion adsorbent treatment is preferably performed by a batch method.

When the metal ion adsorbent is a cation exchange resin, it is preferable to equilibrate the resin to a pH near neutral before treatment.

The metal ion adsorbent treatment is performed at a temperature and CO₂ concentration suitable for the survival and proliferation of cells. The treatment temperature is, for example, about 30°C to about 40°C, preferably about 37°C. The CO₂ concentration is, for example, about 1% by volume to about 10% by volume, preferably about 5% by volume. However, since the treatment is performed in a short time, the temperature and CO₂ concentration may be outside the above-mentioned ranges as long as the cells are not adversely affected.

The vehicle for the metal ion adsorbent treatment is not particularly limited as long as it does not adversely affect the survival of the cells constituting the cell aggregate. Examples of the vehicle include buffer solutions and cell culture mediums (hereinafter also to be simply referred to as "culture medium"). Considering the use in the present invention, in which the single-celled cells after treatment are reseeded and passaged, a culture medium that can continuously maintain the cells is preferably used as the vehicle.

Examples of the "buffer" include buffer solutions of phosphate, acetate, carbonate, citrate, and the like.

The culture medium is, for example, a basal medium to which various components necessary for the survival and proliferation of the target cells have been added.

In the present specification, "basal medium" refers to a medium containing carbon sources, nitrogen sources, inorganic salts, and the like, which are essential for culturing cells. The basal medium that can be used as a component of the medium of the present invention is not particularly limited, and may be selected appropriately according to the type of cells to be cultured. The basal medium may be prepared by a method known per se, or a commercially available product may be used.

Examples of basal media that can be used include Dulbecco's Modified Eagle's Medium (DMEM), Ham's Nutrient Mixture F12, DMEM/F12 medium, McCoy's 5A medium, Minimum Essential Medium (MEM), Eagle's Minimum Essential Medium (EMEM), alpha Modified Eagle's Minimum Essential Medium (αMEM), Roswell Park Memorial Institute (RPMI) 1640 medium, Iscove's Modified Dulbecco's Medium (IMDM), MCDB131 medium, William's Medium E, Fischer's Medium, and the like.

Furthermore, basal media used particularly for stem cells (particularly pluripotent stem cells) include STEMPRO (registered trademark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trademark) Serum-Free Medium for hES cells (Millipore), PluriSTEM (trademark) Human ES/iPS Medium (EMD Millipore), NutriStem (registered trademark) hESC XF medium (Biological Industries Israel Beit-Haemek), NutriStem (trademark) XF/FF Culture Medium (Stemgent), AF NutriStem (registered trademark) hESC XF medium (Biological Industries Israel Beit-Haemek), S-medium (DS Pharma Biomedical Co., Ltd.), StemFit (registered trademark) AK03N medium (Ajinomoto Co., Inc.), hESF9 medium, hESF-FX medium, CDM medium, DEF-CS 500 Xeno-Free 3D Spheroid Culture Medium (Cellartis), StemFlex medium (Thermo Fisher Scientific), and the like.

Furthermore, components that are favorable for cell proliferation can also be added in addition to the basal medium. Such components include, for example, sugars such as glucose, fructose, sucrose, maltose, etc.; amino acids such as asparagine, aspartic acid, glutamine, glutamic acid, etc.; proteins such as albumin, transferrin, etc.; peptides such as glycylglycylglycine, soybean peptide, etc.; serum; vitamins such as choline, vitamin A, vitamin B group (thiamine, riboflavin, pyridoxine, cyanocobalamin, biotin, folic acid, pantothenic acid, nicotinamide, etc.), vitamin C, vitamin E, etc.; fatty acids such as oleic acid, arachidonic acid, linoleic acid, etc.; lipids such as cholesterol, etc.; inorganic salts such as sodium chloride, potassium chloride, calcium chloride, magnesium sulfate, sodium dihydrogen phosphate, etc.; trace elements such as zinc, copper, selenium, etc.; buffers such as N,N-bis(2-hydroxyethyl)-2-aminoethanesulfonic acid (BES), 4-(2-hydroxyethyl)-1-piperazineethanesulfonic acid (HEPES), N-[tris(hydroxymethyl)methyl]glycine (Tricine), etc.; antibiotics such as amphotericin B, kanamycin, gentamicin, streptomycin, penicillin, etc.; cell adhesion factors and extracellular matrix components such as Type I collagen, Type II collagen, fibronectin, laminin, poly-L-lysine, poly-D-lysine, etc.; cytokines and growth factors such as interleukin, fibroblast growth factor (FGF), hepatocyte growth factor (HGF), transforming growth factor (TGF)-α, transforming growth factor (TGF)-β, vascular endothelial growth factor (VEGF), activin A, etc.; and hormones such as dexamethasone, hydrocortisone, estradiol, progesterone, glucagon, insulin, etc., and appropriate components can be selected and used according to the type of cells to be cultured.

### 3. Cell culture method

The present invention provides a cell culture method (hereinafter also to be referred to as the "culture method of the present invention"), which includes the following steps:
(1) a first step of preparing a cell aggregate;
(2) a second step of dissociating the cell aggregate obtained in the first step by a treatment with a water-insoluble metal ion adsorbent in a vehicle; and
(3) a third step of culturing the dissociated cells obtained in the second step.

### <The first step>

This step can be performed by a method known per se, and may be performed under adherent culture conditions or under suspension culture conditions.

### (Under adherent culture conditions)

Cell aggregates prepared under adherent culture conditions include, for example, adherent cell populations resulting from the growth of adherent cells while adhering to each other and sufficient growth of cells while adhering to the surface of a culture substrate, and colonies formed by cells closely adhered to each other. A step for passage is required because aggregation causes a shortage of oxygen and nutrients to be supplied to each cell, which lowers the proliferation rate.

Culture substrates include, but are not limited to, multi-well plate, culture dish, petri dish, culture flask, microcarrier, hollow fiber, and the like. Cultivation may be performed not only by static culture on a substrate, but also by circulation of the medium. For example, culture using a microcarrier as a substrate includes culturing cells attached to microcarriers by suspending and stirring them in a culture medium.

### (Under suspension culture conditions)

Cell aggregates (spheres) prepared under suspension culture conditions are cell aggregates obtained by, for example, a method using a container with a cell non-adhesive surface, hanging drop method, gyratory culture method, stirring culture method, rotation culture, three-dimensional scaffold method, centrifugation method, or a method using aggregation by an electric field or a magnetic field. Alternatively, it is a cell population obtained by culturing in a spherical or fibrous carrier made of alginate gel or the like. Either method can be performed in accordance with the method generally performed in this field. A mixture of the target cells with a culture medium is prepared, and the mixture is seeded in a sterile container suitable for cell culture and cultured for several days to form a sphere. The material of the surface of the culture vessel used at this time is not questioned. For example, it may be a cell adhesive surface or a surface that has been treated for non-adhesion. In the gyratory culture method, cells are seeded in a dish, flask, cell bag, etc., and cultured while circulating the culture medium with an orbital shaker or rocking system, etc., and the cells spontaneously form a sphere within a few hours to one day. In the stirring culture method, cells are seeded in a culture vessel equipped with stirring blades such as a spinner flask, and when the culture is performed while stirring the culture medium using a magnetic stirrer or the like, the cells spontaneously form spheres within a few hours to one day.

In the first step of the present invention, cell aggregates are preferably prepared under suspension culture conditions.

### <The second step>

This step is a step of dissociating the cell aggregates obtained in the first step, by treating them with a water-insoluble metal ion adsorbent in a vehicle, and can be performed in the same manner as in the aforementioned "dissociation method of the present invention".

It is preferable to perform the treatment under conditions continuous with the first step, and for example, when dissociating cell aggregates formed during suspension culture, it is performed under suspension culture conditions. Specifically, it is performed by adding the dissociation agent of the present invention to a cell suspension containing the cell aggregates under suspension culture conditions. When dissociating cell aggregates formed during adherent culture, it is performed by adding the dissociation agent of the present invention to the cell culture substrate on which the cell aggregates are formed.

### <The third step>

This step is a step of culturing the dissociated cells obtained in the second step. This step involves recovering the dissociated cells, reseeding them, and culturing them. The second step to the third step can also be called passage steps.

The culture may be any of adherent culture and suspension culture. The culture can be high-density culture. It is preferably a step including suspension culturing and forming cell aggregates again.

The dissociated (single-celled) cells obtained in the second step can be recovered by a conventional method such as filtration, centrifugation and the like. In the present invention, a water-insoluble sedimentary cation exchange resin (metal ion adsorbent) is used, and the cation exchange resin can be sedimented by simply allowing the resin to stand for several seconds to several tens of minutes, preferably within 30 min, more preferably within 10 min, without performing filtration or centrifugation, and the single-celled cells present in the solution portion can be recovered by pipetting or decantation. In addition, when the cation exchange resin (metal ion adsorbent) is housed in a member that the metal ion adsorbent cannot permeate, and used, the member is left in the medium containing the cell aggregates for a certain period of time, for example, several seconds to several tens of minutes, preferably within 30 min, more preferably within 10 min (standing still is not necessary), and then the member is pulled up and the single-celled cells present in the solution portion can be recovered by pipetting or decantation. Examples of such member include bag (pack)-shaped members made of mesh or membrane having a pore size smaller than that of the metal ion adsorbent, and small devices (e.g., filter device) equipped with the mesh or membrane. The shape of the member is not particularly limited as long as it has a portion where the vehicle containing the cell aggregates and the metal ion adsorbent can come into contact with each other and is large enough to be immersed in the vehicle. Even when a metal ion adsorbent molded into a bulk shape is used, the bulk can be left in the vehicle containing the cell aggregates for a certain period of time, for example, from a few seconds to a few tens of minutes, preferably within 30 min, more preferably within 10 min, and then the bulk can be pulled up and the single-celled cells present in the solution portion can be recovered by pipetting or decantation.

When a culture medium is used as the vehicle, the recovered cells are subjected to adherent culture or suspension culture as they are or in a new culture medium.

In the present specification, "adherent culture" means culturing while adhering to a culture substrate, specifically a method of growing adherent cells while adhering to the surface of the culture substrate and while adhering to each other. Examples of the culture substrate include, but are not limited to, multi-well plate, culture dish, petri dish, culture flask, microcarrier, hollow fiber, and the like. The culture may be a stationary culture on the substrate. In addition, the medium may be circulated during the culture. For example, in culture using a microcarrier as a substrate, a method of culturing while suspending and stirring the microcarrier, to which the cells are attached, in the culture medium is also included in the adherent culture.

In the present specification, "suspension culture" means a cell culture method which is performed in a state in which the cells are not adhered to the culture vessel. In the present invention, suspension culture may or may not accompany external pressure or vibration on the liquid medium, or shaking or rotation in the liquid medium.

For example, a method using a container with a cell non-adhesive surface, hanging drop method, gyratory culture method, stirring culture method, rotation culture, three-dimensional scaffold method, centrifugation method, a method using aggregation by an electric field or a magnetic field, and the like can be mentioned. Alternatively, a method including culturing in a spherical or fibrous carrier made of alginate gel or the like can be mentioned. Either method can be performed in accordance with the method generally performed in this field.

In the present specification, "high density culture" refers to culture at a cell density higher than the cell density (normal density) assumed in general cell culture. The standard for high density may vary depending on the culture method (adherent culture/suspension culture) and the type of cell. For example, in the case of suspension culture of iPS cells, culture at a density of 6×10⁵ cells/mL or more (preferably 2×10⁶ cells/mL or more) is exemplified in the present specification. One embodiment that can be suitably applied is high-density culture using a bioreactor or the like.

Culture conditions are not particularly limited, and a method known per se may be selected according to the cell type, cell density (normal density/high density), culture method (adherent culture/suspension culture, etc.), and the like. For example, culture temperature may be generally 25°C to 39°C, preferably 33°C to 39°C. The CO₂ concentration may be generally 4% to 10% by volume, preferably 4% to 6% by volume. The oxygen concentration may be generally 1% to 25% by volume, preferably 4% to 20% by volume.

The frequency of medium exchange in cell culture is generally determined by comprehensively taking into account various conditions such as cell density (normal density/high density), culture method (adherent culture/suspension culture), type of cell to be cultured, composition of medium, culture conditions (temperature, gas concentration), amount of medium to be exchanged (total amount/partial amount), cost of medium, lifestyle of the worker, and the like. The frequency is generally once every 2-3 days, once a day, or multiple times (e.g., twice) a day, and medium exchange can also be performed at such frequencies in the method of the present invention. The medium exchange may involve replacing the entire amount of the medium in use, or may involve replacing only a portion thereof (e.g., 10%, 20%, 30%, 40%, 50%, 60%, 70%, 80%, or 90% of the total amount of the medium in use).

Depending on the type of cells to be cultured, cells are passaged when the number thereof has reached a certain level or more, generally about 80% or more confluent. The dissociation agent and dissociation method of the present invention can also be used for such passage.

The present invention is described in more detail in the following by illustrating examples, but the scope of the present invention is not limited by these examples.

### [Example]

### Example 1: Dissociation of human iPS cell aggregates into single cells using weakly acidic cation exchange resin, and passage thereof

### (1) Sterilization and pH equilibration of ion exchange resin (WK11)

Into a heat-resistant medium bottle were added weakly acidic cation exchange resin (Mitsubishi Chemical Corporation, Diaion^{™} WK11) (2 g) and D-PBS(-) (50 mL), and the mixture was sterilized by autoclaving at 121°C for 20 min. The sterilized resin was transferred to a 50-mL conical tube, and D-PBS(-) (50 mL) was added. A small amount of 5N sodium hydroxide was added thereto, and the conical tube was slowly inverted repeatedly to mix and adjust the pH of the supernatant to 7.0. After confirming that the pH reached full equilibrium, the supernatant was removed to obtain a pretreated resin (hereafter referred to as "WK11").

### (2) Maintenance culture of iPS cell

To a culture vessel precoated with iMatrix-511 (nippi) was added StemFit (registered trademark) AK03N (Ajinomoto Co., Inc.) containing 10 µM Y-27632 (Wako, 036-24023), and human iPS cells (iPS Academia Japan, Inc., 1383D2 strain) were seeded. Static culture was performed in a CO₂ incubator at 37°C, and the medium was exchanged once every 1 to 3 days with AK03N not containing Y-27632. Seven days after seeding, the cells were detached using CTS^{™} TrypLE^{™} Select (Gibco^{™}) and reseeded in a new culture vessel.

### (3) Suspension culture

The iPS cells maintenance cultured in (2) were seeded at a concentration of 1.5×10⁵ cells/mL in a multi-well plate with StemFit (registered trademark) AK03N (Ajinomoto Co., Inc.) containing 10 µM Y-27632 added thereto, and cultured with stirring at a rotation speed of 70 rpm using an orbital shaker MaxQ^{™} 2000 CO₂ Plus (Thermo Fisher Scientific) placed in a CO₂ incubator at 37°C. One and three days after seeding, 50 vol% each of the medium was exchanged with AK02N, and the culture was continued until four days later to obtain cell aggregates.

### (4) Dissociation of cell aggregates by WK11

To a culture medium containing the cell aggregates prepared in (3) were added Y-27632 (final concentration 10 µM) and WK11 pre-treated in (1), and the mixture was stirred for 17 min using an orbital shaker. Thereafter, the cell aggregates were sufficiently brought to a single-cell state by pipetting, and the viable cell density was measured using a live/dead cell autoanalyzer Vi-CELL XR (BECKMAN COULTER).

### (5) Dissociation of cell aggregates by EDTA (without centrifugation)

To a culture medium containing the cell aggregates prepared in (3) were added Y-27632 (final concentration 10 µM) and 0.5 mol/L ethylenediaminetetraacetic acid solution (Nacalai Tesque Inc., 06894-14, hereafter referred to as "EDTA") adjusted to pH 8.0 to a final concentration of 2.5 mM, and the mixture was stirred for 14 min using an orbital shaker. Thereafter, the cell aggregates were sufficiently brought to a single-cell state by pipetting, and the viable cell density was measured using Vi-CELL XR.

### (6) Dissociation of cell aggregates using EDTA (with removal by centrifugation)

To a culture medium containing the cell aggregates prepared in (3) were added Y-27632 (final concentration 10 µM) and EDTA (final concentration 2.5 mM), and the mixture was stirred for 14 min using an orbital shaker. The cell aggregates were sufficiently brought to a single-cell state by pipetting, the supernatant was removed by centrifugation (200G, 5 min, 25°C), and the mixture was resuspended in AK02N containing 10 µM Y-27632. The viable cell density of the obtained cell suspension was measured using Vi-CELL XR.

### (7) Suspension culture after passage

A new multi-well plate containing AK02N containing 10 µM Y-27632 was prepared, and the cells obtained in (4), (5), and (6) were respectively seeded at a density of 1.5×10⁵ cells/mL. The cells were cultured in a CO₂ incubator while stirring with an orbital shaker. One and three days after seeding, 50 vol% each of the medium was exchanged with AK02N, and the suspension culture was continued until four days later.

To accurately measure the number of viable cells, after four days of culture, under the same conditions, the following operation, including centrifugation, was performed. The entire volume of each culture medium was collected in a 15-mL conical tube, and the supernatant was removed by centrifugation (200 G, 5 min, 25°C). Accutase^{™} (Nacalai Tesque Inc., 12679-54) (0.5 mL) was added and the mixture was heated at 37°C for 15 min, after which the cell aggregates were sufficiently brought to a single-cell state by pipetting. The cells attached to the wall of the conical tube were sufficiently washed with AK02N, and the supernatant was removed by centrifugation (200 G, 5 min, 25°C). The cells were resuspended in AK02N containing 10 µM Y-27632, and the viable cell density was measured using Vi-CELL XR.

### (8) Optical micrographs

Fig. 1 shows a micrograph of the cell aggregates after 4 days of suspension culture produced in (3). Fig. 2 shows micrographs taken immediately after the addition of WK11 and after 17 min of stirring in (4), and Fig. 3 shows micrographs taken immediately after the addition of EDTA and after 14 min of stirring in (5) and (6). Immediately after the addition of WK11 and EDTA, the cell aggregates were highly dense and spherical, but after stirring, they were fragmented and the intercellular binding was weakened to the degree that they could be easily dissociated into single cells by pipetting.

Fig. 4 shows micrographs of cells that were passaged under each condition and further suspension cultured for 4 days. The cells passaged using WK11 were observed to have proliferated sufficiently at the second passage. On the other hand, the cells passaged using EDTA proliferated hardly. In particular, no aggregates were formed under conditions without a step of removing EDTA by centrifugation.

### (9) Viable cell density

The viable cell density in the culture medium was calculated from the Vi-CELL measured values in Example 1 (4), (5), and (6). The results are shown in Fig. 5. A great difference in the viable cell density was not observed depending on the passage method. The viable cell density in the culture medium was calculated from the Vi-CELL measured values in Example 1(7). The results are shown in Fig. 6. The cells passaged using WK11 proliferated 3.4-fold in 4 days, whereas the cells passaged using EDTA without centrifugation removal proliferated 0.03-fold. Even when the centrifugation step was performed, the value was as low as 0.4-fold.

### (10) Undifferentiated marker positive rate

The undifferentiated rate was evaluated for cells 4 days after suspension culture seeding, according to the following procedure.

1×10⁵ cells were quantitatively collected, centrifuged (400 g, 5 min), and the supernatant was removed. 20 µL of PE Mouse Anti-Human CD30 (BD Pharmingen^{™}) or PE Mouse IgG1, κ Isotype Control (BD Pharmingen^{™}) diluted 5-fold with buffer was added and the cells were left standing for 20 min at 4°C in the dark. After washing several times with buffer, the cells were analyzed using an Attune NxT Flow Cytometer (Thermo Fisher Scientific). The analyzed undifferentiated marker (CD30) positive rate is shown in Fig. 7. When single cells were generated using either WK11 or EDTA, the undifferentiated marker maintained high values (Passage 1), and it was shown that they were maintained even after culture (Passage 2). However, measurement was not performed under conditions without a step of removing EDTA by centrifugation, because cells were not obtained.

### [Industrial Applicability]

According to the present invention, cell aggregates, particularly cell aggregates suspended in culture medium, can be efficiently dissociated, and the dissociated cells can be collected without a centrifugation treatment and can be suitably used for passaging. As a result, more cells, particularly pluripotent stem cells such as iPS cells, can be obtained, and the cells can be supplied in large quantities for use in research, medicine, and the like.

This application is based on a patent application No. 2023-008929 filed in Japan (filing date: January 24, 2023), the contents of which are incorporated in full herein.

## Claims

1. A method for dissociating a cell aggregate, comprising subjecting the cell aggregate to a treatment with a water-insoluble metal ion adsorbent in a vehicle.

2. The method according to claim 1, wherein the metal ion adsorbent has sedimentation property.

3. The method according to claim 1, wherein the metal ion adsorbent is housed in a member impermeable to metal ion adsorbents.

4. A method for culturing cells, comprising the following steps:
a first step of preparing a cell aggregate;
a second step of dissociating the cell aggregate obtained in the first step by a treatment with a water-insoluble metal ion adsorbent in a vehicle;
and a third step of culturing the dissociated cells obtained in the second step.

5. The method according to claim 4, wherein the metal ion adsorbent has sedimentation property.

6. The method according to claim 4, wherein the metal ion adsorbent is housed in a member impermeable to metal ion adsorbents.

7. The method according to claim 1 or 4, wherein the metal ion adsorbent has cation exchange property.

8. The method according to claim 1 or 4, wherein the metal ion adsorbent is a weakly acidic cation exchange resin.

9. The method according to claim 1 or 4, wherein the metal ion is Ca²⁺ and/or Mg²⁺.

10. The method according to claim 1 or 4, wherein the cell is a stem cell or a progenitor cell.

11. The method according to claim 10, wherein the stem cell is a pluripotent stem cell or an adult stem cell.

12. The method according to claim 11, wherein the pluripotent stem cell is an induced pluripotent stem cell (iPS cell) or an embryonic stem cell (ES cell).

13. The method according to claim 11, wherein the adult stem cell is a mesenchymal stem cell.

14. The method according to claim 4, wherein the first step is performed under adherent culture conditions.

15. The method according to claim 4, wherein the first step is performed under suspension culture conditions.

16. The method according to claim 4, wherein the second step is performed under adherent culture conditions.

17. The method according to claim 4, wherein the second step is performed under suspension culture conditions.

18. The method according to claim 4, wherein the third step is performed under adherent culture conditions.

19. The method according to claim 4, wherein the third step is performed under suspension culture conditions.

20. The method according to claim 1 or 4, wherein cell aggregate dissociation is inhibition of cell-cell adhesion.

21. The method according to claim 1 or 4, wherein cell aggregate dissociation is inhibition of cell-substrate adhesion.

22. A cell aggregate dissociation agent comprising a water-insoluble metal ion adsorbent.

23. The dissociation agent according to claim 22, wherein the metal ion adsorbent has sedimentation property.

24. The dissociation agent according to claim 22, wherein the metal ion adsorbent is housed in a member impermeable to metal ion adsorbents.

25. The dissociation agent according to claim 22, wherein the metal ion adsorbent has cation exchange property.

26. The dissociation agent according to claim 22, wherein the metal ion adsorbent is a weakly acidic cation exchange resin.

27. The dissociation agent according to claim 22, wherein the metal ion is Ca²⁺ and/or Mg²⁺.

28. The dissociation agent according to claim 22, wherein the cell is a stem cell or a progenitor cell.

29. The dissociation agent according to claim 28, wherein the stem cell is a pluripotent stem cell or an adult stem cell.

30. The dissociation agent according to claim 29, wherein the pluripotent stem cell is an induced pluripotent stem cell (iPS cell) or an embryonic stem cell (ES cell).

31. The dissociation agent according to claim 29, wherein the adult stem cell is a mesenchymal stem cell.
